# EUROPEAN PATENT SPECIFICATION

(11) **EP 2 173 499 B1**
(45) Date of publication and mention of the grant of the patent: **16.03.2011**
(21) Application number: 08790020.5
(22) Date of filing: 26.06.2008
(51) Int. Cl.: B06B 1/06, A61H 23/02

(54) **ACTUATOR WITH INTERCHANGEABLE BENDING-VIBRATING FOILS, COMPRISING A LANGEVIN-TYPE TRANSDUCER**
AKTUATOR MIT AUSTAUSCHBAREN BIEGUNGS- UND VIBRATIONSFOLIEN, AUFWEISEND EINEN LANGEVIN-WANDLER
ACTIONNEUR AVEC DES FEUILLES FLÉCHISSANTES-VIBRANTES INTERCHANGEABLES ET COMPRENANT UN TRANSDUCTEUR DE TYPE LANGEVIN

(30) Priority: 27.07.2007 IT SA20070025
(43) Date of publication of application: 14.04.2010
(73) Proprietor: Universita' Degli Studi di Salerno, 84084 Fisciano (SA) (IT)
(72) Inventor: LAMBERTI, Nicola Antonio, I-84084 Fiscinao (SA) (IT); CASACCHIA, Giuseppe, I-84084 Fisciano (SA) (IT); ARDIA, Luca, I-84084 Fisciano (SA) (IT); CAPUTO, Andrea, I-84084 Fisciano (SA) (IT)
(74) Representative: Santi, Filippo
(86) International application number: PCT/IT2008/000431
(87) International publication number: WO 2009/016673

(56) References cited:
- JP-A- 6 210 244
- JP-A- 2006 082 060
- US-A- 5 406 531

## Description

The present invention concerns an actuator with interchangeable bending-vibrating foils, based on a Langevin-type transducer.

More in detail, the present invention refers to a piezoelectric actuator able to excite a bending motion in a steel foil; the overall system has a high efficiency, low losses and high mechanicals stiffness. The applications of the device are mainly in aesthetics and dermatology.

The typology of piezoelectric actuators able to excite a bending motion in a steel foil is known.

The applications of the device are in dermatological aesthetics and in particular it can be used for:
- Exfoliation of the most superficial horny layer (for example dead cells, sebum);
- Bacterial sterilisation (causing spots and acne);
- Increase of the blood sprinkling;
- Stimulation of the cell turnover;
- Moisturising;
- Elimination of the non-pathological brown blots of the superficial layers (for example cloasma);
- Rebalancing of the local metabolism;
- Removal of the wrinkles;
- Removal of the black points.

Efficiency of the treatment is obtained by making the steel foil vibrating at the frequency of around 25 kHz.

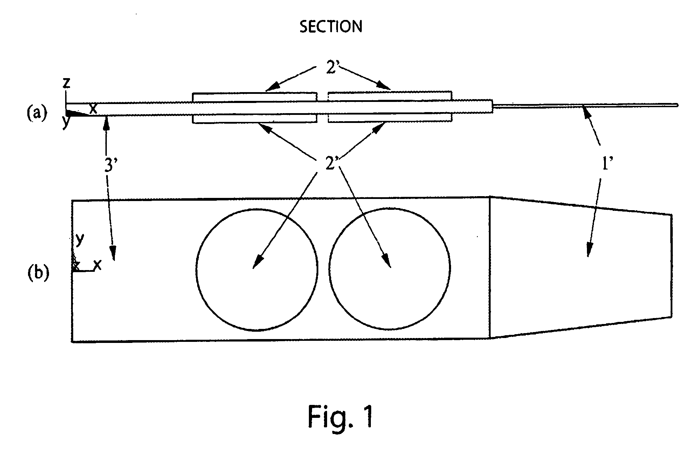

To make the foil vibrating, the current state of the art provides for the use of the device shown in figure 1. The device is constituted by the following parts:
- Vibrating steel foil 1';
- Four piezoelectric ceramics 2'; and
- Steel base 3'.

The motion is induced by exploiting the radial mode of the piezoelectric ceramics 2': the ceramics placed on the upper surface of the base 3' are excited by a sinusoidal signal which is in counterphase with respect to those placed on the lower surface, thus forcing the base itself to bend; and this motion is therefore transferred to the vibrating foil 1'.

The excitation of a transversal mode in the piezoelectric ceramics, i.e. a mode in which the direction of the motion is perpendicular to that of the applied electrical field, is exactly one of the problems of the actuator shown in figure 1: the transversal modes show indeed a transduction efficiency lower than that of longitudinal ones, that is those in which the motion direction is parallel to that of the applied electrical field.

Another problem is represented by the way by which the foil 1' is bound to the base 3': in the current device the joint is realised by brazing; in the actuator functioning exactly this one revealed as the weakest part: it has been observed indeed a yielding, followed by a detachment of the foil 1' of the base 3'; this phenomenon considerably reduces the useful life of the device.

Further, the stress undergone by the device of the prior art can lead to the depolarisation of the piezoelectric ceramics, as well as their detachment (they are only glued to the base).

Finally, the fact that the transducer and the foil are a unique element excludes the possibility to change the foil with respect to the zones of the body to be treated.

It is object of the present invention to provide a piezoelectric actuator able to excite a bending motion in a steel foil which solves partially or totally the problems of the prior art and avoids the above described disadvantages.

It is subject-matter of the present invention an actuator with bending-vibrating foils, comprising:
- a transducer of the "Langevin" type, connected to a
- foil with a longitudinal principal extension direction, characterised in that:
- the transducer is such to excite longitudinal modes, i.e. modes in which the direction of the produced motion is equal to that of the applied electrical field;
- foil is placed in such a way that its longitudinal principal extension direction is substantially perpendicular to the direction of the motion produced by said transducer.

According to the invention, the connection between the transducer and a foil is realised by means of a support that has a superficial dimensional substantially equal to the cross-section of the end of the transducer which connects to the foil and the other superficial dimensions equal to the width of the foil at the connection zone. According to the invention, said support shows a threaded aperture and the end of the transducer which connects to the foil presents a pin for realising such a connection together with the bolt placed on the support, so as to lower the stresses on such joint and allow the simple substitution by the user of the whole constituted by foil and support.

Preferably according to the invention, the foil and the support are formed as an only part.

Preferably according to the invention, said transducer has an extended form and comprises a head and a tail between which piezoelectric elements are placed.

Preferably according to the invention, said head and said tail are made of steel, and said piezoelectric elements are made of ceramics.

Preferably according to the invention, said transducer further comprises a concentrator placed between the foil and the head of the transducer, said concentrator having cross-section smaller than the cross-section of said head.

Preferably according to the invention, the total length of the transducer is substantially equal to one half of the wavelength λ in the material, calculated at the working frequency.

Preferably according to the invention, the thickness of said support is comprised between 1 mm and 5 mm.

Preferably according to the invention, the head is provided with a pin by which it connects to the piezoelectric elements and to the tail which acts as bolt.

Preferably according to the invention, the head and the concentrator are formed as an only piece.

Preferably according to the invention, between the piezoelectric elements thin rings of copper are placed by which the electrical connections between the actuator and an external excitation circuit are realised.

Preferably according to the invention, the electrical contacts are further fixed between themselves and to the head and tail by means of an epoxy resin.

Preferably according to the invention, the piezoelectric elements are four.

Preferably according to the invention, the piezoelectric elements are electrically connected between themselves in parallel and in such a way that the head and the tail are at null potential.

The invention will be now described by way of illustration but not by way of limitation, with particular reference to the figures of the enclosed drawings, wherein:
- Figure 1 shows a scheme of a traditional piezoelectric actuator, cross-section (a) and plant (b);
- Figure 2 shows the distribution of stresses intensity in the traditional device according to figure 1, calculated by simulation at working frequency (23.6 kHz);
- Figure 3 shows a scheme of an embodiment of the piezoelectric actuator according to the invention;
- Figure 4 shows the distribution of the displacements in the piezoelectric actuator according to figure 3, calculated by simulation at the frequency of 26,1 kHz;
- Figure 5 shows the distribution of the stresses in the piezoelectric actuator according to figure 3, calculated by simulation at working frequency (26,1 kHz).

The research of a new solution of piezoelectric actuator with bending-vibrating foils has been preceded by researches on the mechanical stability of the prior art solution.

In order to verify whether the fragility of the prior art device (figure 1) is due to structural problems or to a bad realisation thereof, its functioning has been simulated by means of ANSYS code which implements a finite-elements analysis method (FEM).

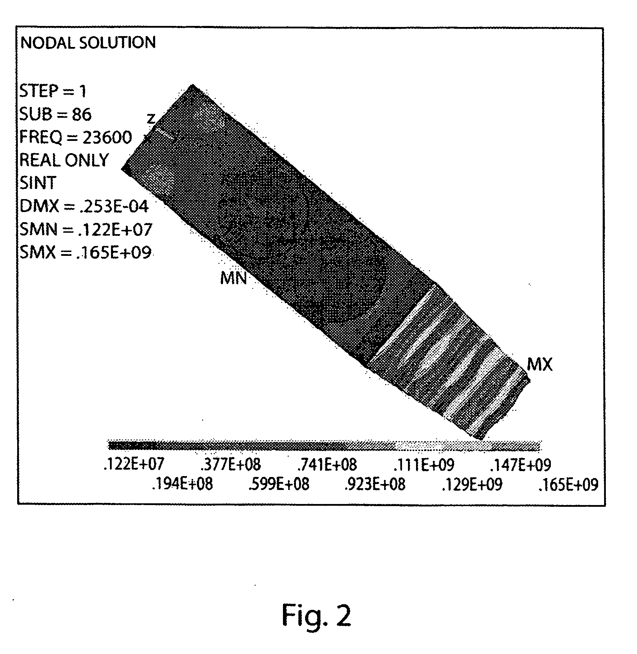

The results obtained by means of the FEM analysis are shown in figure 2 and highlighted the maximum of the mechanical stress exactly at the joint between the base 3' and foil 1'. The stress, in this zone, calculated by imposing a power supply voltage with an amplitude equal to 250 V (typical for those applications), varies, depending on the excitation frequency, between 120 and 150 MPa and is of the same magnitude order of the yield point, of around 500 MPa for the steel of which base and foil are constituted.

Finally, the fact that the joint is realised by brazing further increases the risk of breaking; in such a case indeed the yield point is very below of that of the material.

One can therefore conclude that the reason of the short life of the prior art device is implied in its configuration and therefore is of structural type.

In figure 2 regions can be observed as well, at the boundary between the ceramics and the base, which are also interested by a high stress, between 1 and 20 MPa; the causes are to be mainly attributed to a series of natural sub-modes, which superpose to the interest principal mode, and so that the ceramics undergo light lateral torsions which result in higher stress on the same. The negative consequence of this high value of the stress is an excessive stress of the glue by which the ceramics are bound to the base, with following detachment of the ceramics themselves, a phenomenon which occurred on some samples of the actuator.

Finally, always in figure 2, in the middle of the ceramics farthest from the foil, one notices a stress that can reach even 37 MPa, that is to exceed the limit value (30 MPa) beyond which the ceramics depolarise and therefore loose the piezoelectric properties, thus making the device as ineffective.

The device according to the present invention exploits the action of the piezoelectric ceramics in a way completely different with respect to the current device.

In particular, the bending motion of the foil is induced by exciting the ceramics in the thickness mode, which, being a longitudinal mode, allows to obtain a better transduction efficiency.

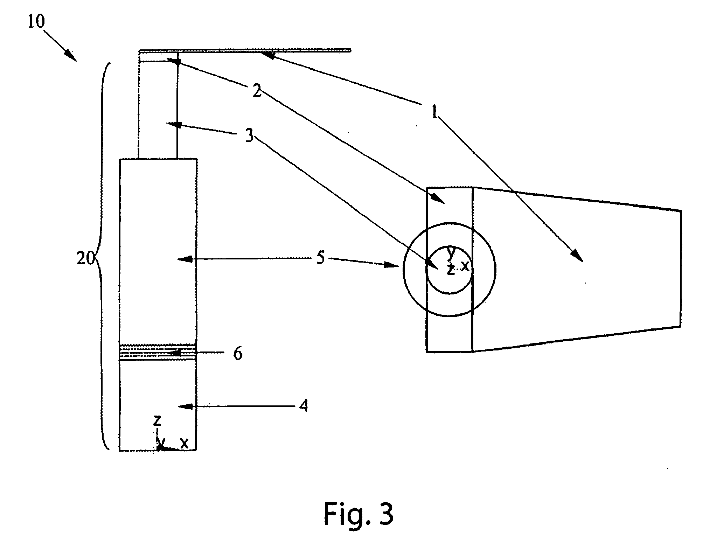

The proposed device is shown in figure 3; is constituted by the following products:
- Vibrating steel foil 1;
- Steel supports 2 of the foil;
- Step concentrator 3 of the Langevin actuator, made of steel;
- Tail 4 of the Langevin actuator, made of steel;
- Head 5 of the Langevin actuator, made of steel;
- Piezoelectric ceramics 6.

The parts 3, 4, 5 and 6 constitute a transducer or piezoelectric actuator of the Langevin type, complete of concentrator 3. With the term "actuator" is meant also the whole of the illustrated device.

The support 2 is necessary in order to transfer the motion from transducer 20 to foil 1; for this reason its width is equal to that of the foil 1, whilst the length is equal to the diameter of the concentrator 3.

The design parameters of the actuator according to the invention are the longitudinal dimension of the body of the transducer (tail 4, head 5 and piezoelectric ceramics 6), the ratio between the length of the tail 4 and of the head 5, the ratio between the radius of the head 5 and that of the concentrator 3. The length of the body of the transducer determines its resonance frequency, the number and the dimensions of the piezoelectric ceramics, the efficiency of transduction, whilst the ratio between the dimensions of the head and of the tail shifts the zone in which the displacements in the longitudinal direction are maximal. Finally the concentrator has the aim of the amplifying the displacements of the end connected to the foil; the amplification factor is equal to the ratio between the radius of the head and that of the concentrator.

The length of the transducer body (parts 4, 5 and 6) and of the concentrator (part 3) are in general dimensioned so as to be, altogether (and therefore the overall length of the transducer 20), equal to the wavelength (λ) in the material, calculated at working frequency: 25 kHz in the prototype realised by the applicant.

The behaviour of the device has been studied by means of the use of the ANSYS code and it has been verified that if the total length is equal to the half of the wavelength (λ/2), calculated at the working frequency, the amplitude of the displacements of the vibrating foil 1 is larger than that obtained by designing the transducer so that its dimension is equal to λ. By using the λ/2 structure, the actuator behaves better even from an electrical viewpoint, in the sense that the electrical resonance is more pronounced, and therefore one expects a higher efficiency for that solution.

These results allow reducing the longitudinal overall dimensions of the device.

Finally, by means of ANSYS, it has been possible to dimension the thickness of the support 2 of the steel foil so as to transfer in optimised way the motion of the transducer to the foil: for example, with a foil 1 of the thickness of 0,5 mm, the thickness of the support 2 must be of 3 mm. Different dimensions of the support 2 have been verified and the chosen solution allows obtaining a uniform bending motion of the foil.

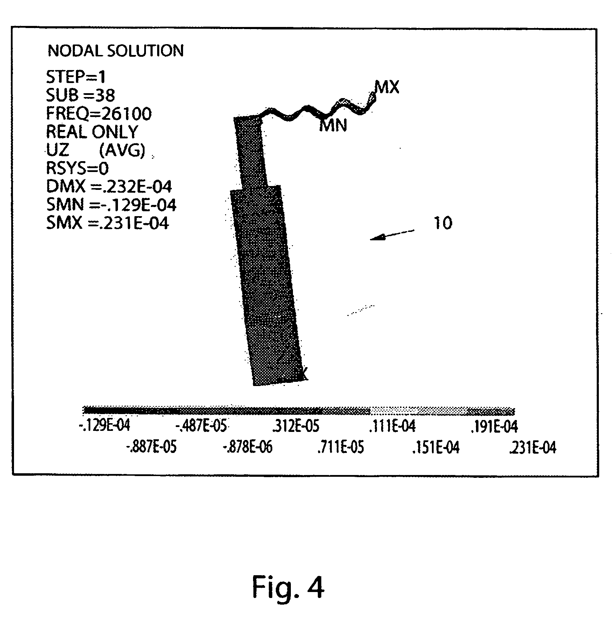

In figure 4 the field of the displacements is shown, calculated by means of ANSYS at a frequency of 26,1 kHz; as already stressed, the "Langevin" transducer has a length equal to λ/2 at that frequency. As one can see, the foil 1 moves uniformly by bending and the displacements have their maximum amplitude exactly at his free end. Further the displacements in the "Langevin" type actuator are negligible with respect to those of the foil, and therefore one expects a good stiffness of the whole structure.

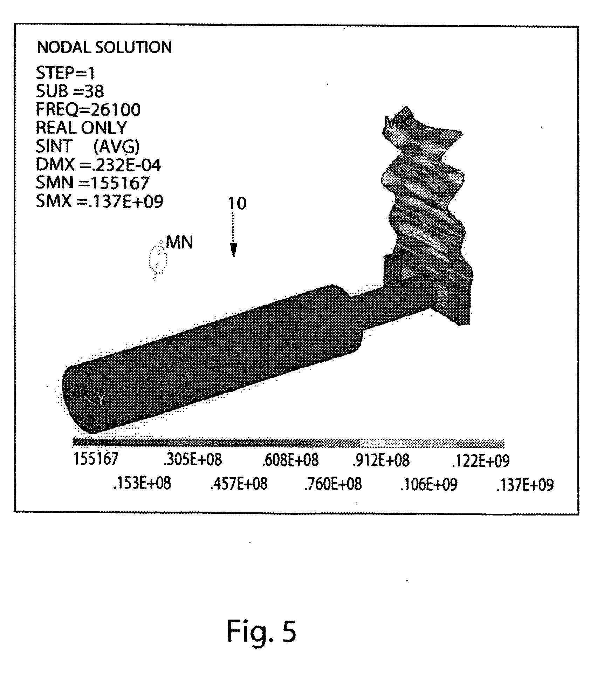

In order to verify whether actually the proposed structure is stiffer than that currently used, also the stresses inside it had been calculated by exciting it at 26,1 kHz, with a sinusoidal voltage of amplitude equal to 250 V. In figure 5 the field of stresses along the structure is shown.

As one can see, only in a small part, in the middle of the contact zone between the foil 1 and a support 2, the stress reaches a value of about 100 MPa, whilst in the remaining part it is below 76 MPa and even sinks below 13 MPa in the external zones. With respect to the traditional solution, the improvement is therefore remarkable.

Even the stress on the piezoelectric ceramics is very small, below 15 MPa; this avoids the depolarisation of the ceramics. Finally, one has the maximum of the stress on the foil, however it doesn't exceed 140 MPa and therefore it is very below the stress limit of the steel.

Concerning the possibility of using vibrating foil with form and dimension different from that shown in figure 3 and in the ANSYS simulations (figures 4 and 5), the solution is very simple, it is sufficient to simply substitute the foil 1 and support 2 with the new foil complete with its own support.

In order to describe even in more detail the practical realisation of the device according to the invention, one makes reference to figure 3, where different parts constituting it are highlighted.

The assembling ways are the following: the vibrating foil 1 and the support 2 are preferably realised in a unique block of harmonic steel and the support has in the middle a hole; further, the upper end of the step concentrator 3 of the actuator has a threaded pin which crosses the support 2 and allows to fix the two parts by means of a bolt placed on the support.

This system to fix the foil to the actuator has the advantage to lower the stresses on the joint, indeed here the foil is not brazed laterally on the support, as in the current device, but superposed to it; it further offers to the user the possibility of easily substituting foil and support, so as to use the foil most suitable to the zone to be treated.

The step concentrator 3 and the head 5 of the actuator realised in a sole part made of steel; as above mentioned, the upper end of the concentrator 3 is provided with a pin in order to be able to fix on it the foil 1 with the support 2, the lower end of the concentrator 3 is also provided with a threaded pin which is screwed on to the tail 4 of the actuator, which in turn acts as a tightening bolt. The lower pin has also the aim of tighten the piezoelectric ceramics 6, which are rings, between the head and the tail of the " Langevin" actuator and of allowing therefore to realise a single device.

Between the ceramics 6, thin rings of copper are placed, with the same surface of the ceramics, by which the electrical connections between the actuator and the excitation circuit are realised.

By means of the mechanical torque by which the head and tail of the " Langevin" are mutually tightened, one sets the pre-stress of the piezoceramics; the pre-stress serves to improve the performances of the whole device.

Finally, the ceramics and the electrical contacts are further bound mutually and to the parts 5 and 4 by means of an epoxy resin, which guarantees a perfect tightening of the structure, avoids possible loosenings due to vibrations, but most of all avoids the presence of air between the various parts of the actuator, what would have dreadful effects on the propagation of the elastic waves in the structure.

Concerning the piezoelectric ceramics, one has chosen to use four ones; as said, this choice influences the efficiency of the actuator, in the sense that the larger is the amount of the ceramics the greater will be efficiency, on the other hand an excessive number of ceramics reduces too much the electrical capacity of the device, therefore it increases its input impedance and make it difficult to supply it from an electrical viewpoint.

Exactly to limit the input electrical impedance of the device, the piezoceramics are connected between them in parallel and in such a way that the parts 4 and 5 in figure 3 are at null voltage; the last arrangement allows to avoid risks of exposure to electrical discharges in the use of the device.

The preferred embodiments of the invention and some of its variations have been hereinabove described, but it should be understood that those skilled in the art can make modifications or changes therein without departing from the scope of this invention as defined by the following claims.

## Claims

1. Actuator with bending-vibrating foil, comprising a transducer (20) of the "Langevin" type, connected to a foil (1) with a longitudinal principal extension direction, such that:
- the transducer (20) is adapted to excite longitudinal modes, i.e. modes in which the direction of the produced motion is equal to that of the applied electrical field;
- the foil (1) is placed in such a way that its longitudinal principal extension direction is substantially perpendicular to the direction of the motion produced by said transducer (20),
- the connection between the transducer (20) and a foil (1) is realised by means of a support (2) that has a superficial dimension substantially equal to the cross-section of the end of the transducer (20) connected to the foil (1) and the other superficial dimension equal to the width of the foil (1) at the connection zone,
the actuator being **characterised in that** said support (2) shows a threaded aperture and the end of the transducer (20) which connects to the foil (1) presents a pin for realising such a connection together with the bolt placed on the support (2), so as to lower the stresses on such joint and allow the simple substitution by the user of the whole constituted by foil (1) and support (2).

2. Actuator according to claim 1, **characterised in that** the foil (1) and the support (2) are formed as an only part.

3. Actuator according to any claim 1 or 2, **characterised in that** said transducer (20) has an extended form and comprises a head (5) and a tail (4) between which piezoelectric elements (6) are placed.

4. Actuator according to claim 3, **characterised in that** said head (5) and said tail (4) are made of steel, and said piezoelectric elements are made of ceramics.

5. Actuator according to claim 3 or 4, **characterised in that** said transducer (20) further comprises a concentrator (3) placed between the foil (1) and the head (5) of the transducer, said concentrator having cross-section smaller than the cross-section of said head (5).

6. Actuator according to any claim 3 to 5, when dependent from claim 3, **characterised in that** the total length of the transducer is substantially equal to one half of the wavelength λ in the material, calculated at the working frequency.

7. Actuator according to any claim 1 to 6, when dependent from claim 2, **characterised in that** the thickness of said support (2) is comprised between 1 mm and 5 mm.

8. Actuator according to any claim 5 to 7, when dependent from claim 6, **characterised in that** the head (5) is provided with a pin by which it connects to the piezoelectric elements (6) and to the tail (4) which acts as bolt.

9. Actuator according to any claim 5 to 8, when dependent from claim 6, **characterised in that** the head (5) and the concentrator (3) are formed as an only piece.

10. Actuator according to any claim 3 to 9, when dependant from claim 4, **characterised in that** between the piezoelectric elements (6) thin rings of copper are placed by which the electrical connections between the actuator (10) and an external excitation circuit are realised.

11. Actuator according to claim 10, **characterised in that** the electrical contacts are further fixed between themselves and to the head (5) and tail (4) by means of an epoxy resin.

12. Actuator according to any claim 3 to 11, when dependent from claim 3, **characterised in that** the piezoelectric elements are four.

13. Actuator according to any claim 3 to 12, when depending from claim 3, **characterised in that** the piezoelectric elements are electrically connected between themselves in parallel and in such a way that the head (5) and the tail (4) are at null potential.

## Patentansprüche

1. Aktor mit Biege-Vibrationsfolie, umfassend einen Transducer (20) des "Langevin"-Typs, verbunden mit einer Folie (1) mit einer Längshauptausdehnungsrichtung, derart dass:
- der Transducer (20) geeignet ist, longitudinale Moden anzuregen, d.h. Moden, bei denen die Richtung der erzeugten Bewegung der des angelegten elektrischen Feldes entspricht;
- die Folie (1) derart angeordnet ist, dass ihre Längshauptausdehnungsrichtung im Wesentlichen senkrecht zu der durch den Transducer (20) erzeugten Richtung der Bewegung ist,
- die Verbindung zwischen dem Transducer (20) und einer Folie (1) durch eine Halterung (2) realisiert ist, welche eine Oberflächenabmessung besitzt, die im Wesentlichen dem Querschnitt des mit der Folie (1) verbundenen Endes des Transducers (20) entspricht und die andere Oberflächenabmessung der Breite der Folie (1) an der Verbindungszone entspricht,
wobei der Aktor **dadurch gekennzeichnet ist, dass** die Halterung (2) eine mit einem Gewinde versehene Öffnung aufweist und das Ende des Transducers (20), das mit der Folie (1) verbunden ist, einen Stift zur Realisierung einer solchen Verbindung zusammen mit dem an der Halterung (2) angeordneten Bolzen aufweist, um die Spannungen bei solch einer Verbindung zu verringern und einen einfachen Austausch des durch Folie (1) und Halterung (2) gebildeten Ganzen durch den Benutzer zu ermöglichen.

2. Aktor nach Anspruch 1, **dadurch gekennzeichnet, dass** die Folie (1) und die Halterung (2) als ein einziges Teil ausgebildet sind.

3. Aktor nach Anspruch 1 oder 2, **dadurch gekennzeichnet, dass** der Transducer (20) eine ausgestreckte Form besitzt und einen Kopf (5) und einen Fuß (4) aufweist, zwischen denen piezoelektrische Elemente (6) angeordnet sind.

4. Aktor nach Anspruch 3, **dadurch gekennzeichnet, dass** der Kopf (5) und der Fuß (4) aus Stahl und die piezoelektrischen Elemente aus Keramik sind.

5. Aktor nach Anspruch 3 oder 4, **dadurch gekennzeichnet, dass** der Transducer (20) weiterhin einen Konzentrator (3) aufweist, der zwischen der Folie (1) und dem Kopf (5) des Transducers angeordnet ist, wobei der Konzentrator einen Querschnitt besitzt, der kleiner als der Querschnitt des Kopfes (5) ist.

6. Aktor nach einem der Ansprüche 3 bis 5 , wenn abhängig von Anspruch 3, **dadurch gekennzeichnet, dass** die Gesamtlänge des Transducers im Wesentlichen der Hälfte der Wellenlänge λ im Material entspricht, berechnet bei der Arbeitsfrequenz .

7. Aktor nach einem der Ansprüche 1 bis 6, wenn abhängig von Anspruch 2, **dadurch gekennzeichnet, dass** die Halterung (2) eine Dicke zwischen 1 mm und 5 mm aufweist.

8. Aktor nach einem der Ansprüche 5 bis 7, wenn abhängig von Anspruch 6, **dadurch gekennzeichnet, dass** der Kopf (5) mit einem Stift versehen ist, durch den er mit den piezoelektrischen Elementen (6) und dem Fuß (4) verbunden ist, der als Bolzen dient.

9. Aktor nach einem der Ansprüche 5 bis 8, wenn abhängig von Anspruch 6, **dadurch gekennzeichnet, dass** der Kopf (5) und der Konzentrator (3) als ein einziges Teil ausgebildet sind.

10. Aktor nach einem der Ansprüche 3 bis 9, wenn abhängig von Anspruch 4, **dadurch gekennzeichnet, dass** zwischen den piezoelektrischen Elementen (6) dünne Ringe aus Kupfer angeordnet sind, durch welche die elektrischen Verbindungen zwischen dem Aktor (10) und einem externen Anregungskreis gebildet werden.

11. Aktor nach Anspruch 10, **dadurch gekennzeichnet, dass** die elektrischen Kontakte ferner untereinander und am Kopf (5) und am Fuß (4) mittels eines Epoxidharzes fixiert sind.

12. Aktor nach einem der Ansprüche 3 bis 11, wenn abhängig von Anspruch 3, **dadurch gekennzeichnet, dass** die Anzahl der piezoelektrische Elemente vier beträgt.

13. Aktor nach einem der Ansprüche 3 bis 12, wenn abhängig von Anspruch 3, **dadurch gekennzeichnet, dass** die piezoelektrischen Elemente untereinander parallel elektrisch verbunden sind und so vorliegen, dass sich der Kopf (5) und der Fuß (4) auf Nullpotential befinden.

## Revendications

1. Actionneur ayant des lames vibrant en flexion, comprenant un transducteur (20) du type "Langevin", connecté à une lame (1) ayant une direction d'extension principale longitudinale, de telle sorte que :
- le transducteur (20) soit adapté pour exciter des modes longitudinaux, à savoir les modes dans lesquels la direction du mouvement produit est identique à celle du champ électrique appliqué,
- la lame (1) soit placée de façon que sa direction d'extension principale longitudinale soit essentiellement perpendiculaire à la direction du mouvement produit par ledit transducteur (20),
- la connexion entre le transducteur (20) et une lame (1) soit réalisée au moyen d'un support (2) qui a une dimension superficielle essentiellement égale à la section transversale de l'extrémité du transducteur (20) connectée à la lame (1) et l'autre dimension superficielle égale à la largeur de la lame (1) au niveau de la zone de connexion,
l'actionneur étant **caractérisé en ce que** ledit support (2) présente une ouverture taraudée et l'extrémité du transducteur (20) qui est connectée à la lame (1) présente un axe pour réaliser cette connexion en association avec le boulon placé sur le support (2), de façon à réduire les contraintes sur cette liaison et à permettre à l'utilisateur d'effectuer un remplacement simple de l'ensemble constitué par la lame (1) et le support (2).

2. Actionneur selon la revendication 1, **caractérisé en ce que** la lame (1) et le support (2) sont formés en une seule pièce.

3. Actionneur selon l'une quelconque des revendications 1 et 2, **caractérisé en ce que** ledit transducteur (20) a une forme allongée et comprend une tête (5) et une queue (4) entre lesquelles sont placés des éléments piézoélectriques (6).

4. Actionneur selon la revendication 3, **caractérisé en ce que** ladite tête (5) et ladite queue (4) sont constituées d'acier, et lesdits éléments piézoélectriques sont constitués de céramique.

5. Actionneur selon la revendication 3 ou 4, **caractérisé en ce que** ledit transducteur (20) comprend en outre un concentrateur (3) placé entre la lame (1) et la tête (5) du transducteur, ledit concentrateur ayant une section transversale plus petite que la section transversale de ladite tête (5).

6. Actionneur selon l'une quelconque des revendications 3 à 5, lorsqu'elles dépendent de la revendication 3, **caractérisé en ce que** la longueur totale du transducteur est essentiellement égale à la moitié de la longueur d'onde λ à l'intérieur du matériau, calculée à la fréquence de travail.

7. Actionneur selon l'une quelconque des revendications 1 à 6, lorsqu'elles dépendent de la revendication 2, **caractérisé en ce que** l'épaisseur dudit support (2) est comprise entre 1 mm et 5 mm.

8. Actionneur selon l'une quelconque des revendications 5 à 7, lorsqu'elles dépendent de la revendication 6, **caractérisé en ce que** la tête (5) est pourvue d'un axe par lequel elle se connecte aux éléments piézoélectriques (6) et à la queue (4) qui joue le rôle d'un boulon.

9. Actionneur selon l'une quelconque des revendications 5 à 8, lorsqu'elles dépendent de la revendication 6, **caractérisé en ce que** la tête (5) et le concentrateur (3) sont formés en une seule pièce.

10. Actionneur selon l'une quelconque des revendications 3 à 9, lorsqu'elles dépendent de la revendication 4, **caractérisé en ce que** de minces bagues de cuivre sont placées entre les éléments piézoélectriques (6), bagues grâce auxquelles sont réalisées les connexions électriques entre l'actionneur (10) et un circuit d'excitation externe.

11. Actionneur selon la revendication 10, **caractérisé en ce que** les contacts électriques sont en outre fixés les uns aux autres ainsi qu'à la tête (5) et à la queue (4) au moyen d'une résine époxy.

12. Actionneur selon l'une quelconque des revendications 3 à 11, lorsqu'elles dépendent de la revendication 3, **caractérisé en ce que** les éléments piézoélectriques sont au nombre de quatre.

13. Actionneur selon l'une quelconque des revendications 3 à 12, lorsqu'elles dépendent de la revendication 3, **caractérisé en ce que** les éléments piézoélectriques sont connectés électriquement les uns aux autres en parallèle et de telle sorte que la tête (5) et la queue (4) soient à un potentiel nul.
